# EUROPEAN PATENT APPLICATION

(11) **EP 4 273 173 A1**
(43) Date of publication of application: **08.11.2023**
(21) Application number: 21912542.4
(22) Date of filing: 18.02.2021
(51) Int. Cl.: C07K 19/00, C12N 15/62, A61K 38/20, A61K 47/64, A61P 35/00

(54) **LONG-ACTING INTERLEUKIN-15 FUSION PROTEIN, PREPARATION METHOD THEREFOR, AND APPLICATION THEREOF**

(30) Priority: 30.12.2020 CN 202011642917
(71) Applicant: Suzhou Forlong Biotechnology Co., Ltd., Suzhou, Jiangsu 215002 (CN)
(72) Inventor: JI, En, Suzhou, Jiangsu 215002 (CN); WANG, Chunyu, Suzhou, Jiangsu 215002 (CN)
(74) Representative: V.O.
(86) International application number: PCT/CN2021/076670
(87) International publication number: WO 2022/141771

(57) **Abstract**

Provided is a long-acting IL-15 fusion protein, comprising a monomeric fragment Fc, an IL-15 receptor α "sushi" domain, and an IL-15 functional fragment. The IL-15 fusion protein has a higher yield than an Fc fusion protein, and can reduce the number of Treg cells in a tumor tissue, effectively activate an immune system to kill tumor cells, and improve the *in vivo* tumor inhibitory effect of IL-15.

## Description

### Cross-Reference to Related Applications

The present application claims the priority of Chinese Patent Application No. 2020116429173 filed with China National Intellectual Property Administration on December 30, 2020, entitled "Long-acting interleukin-15 fusion protein, preparation method therefor and application thereof', the entire content of which is incorporated into the present application by reference.

### Technical Field

The present invention belongs to the field of biotechnology and particularly relates to a long-acting interleukin-15 fusion protein, a preparation method therefor, a nucleic acid, plasmid and host cell required for constructing the long-acting protein, a corresponding pharmaceutical composition, and the use thereof.

### Background Art

IL-15 is a cytokine of about 12-14 kD discovered by Grabstein *et al.* in 1994, which can function in the normal immune response of the body, for example, to promote the proliferation of T cells, B cells and NK cells. Both IL-15 and IL-2 belong to the IL-2 family, and the receptor members of the IL-2 family all contain yc chains, including IL-2, IL-4, IL-7, IL-9, IL-15, and IL-21. Among them, IL-15 and IL-2 share the common IL-2/IL-15Rβ (CD122) and yc receptor (CD132), and the specific receptor of IL-15 is IL-15Rα (CD125).
IL-15 signaling can occur via a heterotrimeric complex of IL-15Rα, IL-15Rβ and yc. In recent years, the mechanism of action of IL-15 is a mechanism of trans-presentation, by which IL-15 and IL-15Rα are co-expressed by APC (monocytes and dendritic cells), and IL-15 bound to IL-15Rα is trans-presented to neighboring NK cells or CD8 T cells that only express IL-15Rβγc receptor. At present, the trans-presentation of IL-15 is the main mechanism of action of IL-15 to play its role *in vivo,* especially to play a major role in the surveillance of tumor immunity.
IL-15Rα contains a Sushi domain, which can bind to IL-15 and is necessary for the bound IL-15 to exert its biological function. However, IL-15 and IL-15Rα are mainly expressed on the surface of dendritic cells and monocytes and are less likely in free state. Therefore, the activation of IL-15 signaling pathway can more likely activate the downstream signaling pathway after cell-to-cell contact. IL-15 and IL-2 have similar functions of activating T cells, but do not activate Treg cells. Therefore, IL-15 will have less side effects in clinical use in the future and may become a surrogate for IL-2. In view of the good prospection of IL-15 in the field of tumor immunotherapies, NIH for the first time conducted research on IL-15 in the treatment of tumors and tried to push it into clinical research. However, due to the small molecular weight and short *in vivo* half-life of natural IL-15, difficulties exist in controlling the repeated dosage and a poor compliance, and it is easy to cause problems such as systemic immune side effects. Therefore, there is an urgent need in the art for an approach to improve the *in vivo* half-life of IL-15 and promote or enhance the biological activity thereof *in vivo.*

At present, there are three long-acting techniques for protein drugs: PEG (polyethylene glycol) modification technique, HSA (human serum albumin) fusion technique, and Fc (human antibody Fc region) fusion technique. These three techniques all have their own weaknesses. Generally, the common key shortcoming is that the molecular weight of the fused or modified protein drug will greatly increase, and the yield and clinical efficacy of the fused protein drug often tends to be significantly reduced. In the field of long-acting proteins, the inventors have innovatively developed a novel monomeric Fc based on an antibody IgG Fc by using synthetic biology (CN 109705211 B; 202011161007.3), which provides a superior alternative for long-term action of protein drugs.

### Summary of the Invention

The main objective of the present application is to construct a long-acting interleukin-15 fusion protein that incorporates a monomeric Fc (sFc), IL-15 and an IL-15 receptor α sushi domain. The fusion protein, which utilizes the mechanism of action of IL-15 trans-presentation, has potentially better developability, longer *in vivo* half-life, and better *in vivo* anti-tumor activity.

In order to achieve the above objective, the present invention provides the following technical solution:
In a first aspect, provided is a long-acting interleukin-15 fusion protein, comprising an Fc monomer, an IL-15 receptor α sushi domain, and an IL-15 functional fragment, wherein one terminal of the IL-15 receptor α sushi domain is linked to the Fc monomer, and the other terminal of the IL-15 receptor α sushi domain is linked to the IL-15 functional fragment.

In a preferred embodiment of the above long-acting interleukin-15 fusion protein, the C-terminal of the IL-15 receptor α sushi domain is linked to the Fc monomer, and the IL-15 receptor α sushi domain is linked to the IL-15 functional fragment via a covalent bond; preferably, the Fc monomer is linked to the IL-15 receptor α sushi domain via a linker peptide, and more preferably, the linker peptide is GGGGS or (GGGGS)₃.

The covalent bond in the present invention refers to a type of chemical bond, wherein two or more atoms share their outer electrons, and in an ideal circumstance, they reach the state of electron saturation to form a relatively stable chemical structure. As such, a strong interaction formed between electrons shared by several adjacent atoms is called a covalent bond.

The linker peptide refers to a polypeptide chain containing a flexible amino acid residue, and the flexible amino acid residue is Gly, Ser, Ala, or Thr. The polypeptide chain should have a suitable distance, which is suitable for linking two molecules such that they have the correct configuration relative to each other so as to maintain the desired activity. Suitable lengths for this purpose include at least one and no more than 30 amino acid residues. Preferably, the length of the linker is about 1-30 amino acids, and a preferred length of the linker is 1, 2, 3, 4, 5, 6, 7, 8, 9, 10, 11, 12, 13, 14, 15, 16, 17, 18, 19, and 20 amino acids. In addition, the amino acid residues selected for inclusion in the linker peptide should not exhibit properties that significantly affect the activities of the two molecules linked. Therefore, the linker peptide generally does not show charges inconsistent with the two linked molecules, or does not affect internal folding, or does not form bonds or other interactions with amino acid residues in one or more monomers, which monomers will seriously hinder the binding of receptor monomer domains. The linker peptide containing flexible amino acid residues include glycine-serine polymers (such as (GS)n, (GSGGS)n, (GGGGS)n, and (GGGS)n, where n is an integer of at least 1), glycine-alanine polymers, alanine-serine polymers, and other flexible linker peptides known in the art, such as the linking sequences of Shaker potassium channels.

The Fc monomer in the present invention refers to such an Fc polypeptide that has a molecular weight only half of that of the wild-type Fc region, retains the FcRn-binding property and Protein A/G binding property of an antibody Fc region, and can realize efficient expression in prokaryotic cells.

In a preferred embodiment of the above long-acting interleukin-15 fusion protein, the sequence of the Fc monomer comprises an amino acid sequence set forth in SEQ ID NO: 1.

X0 is any amino acid selected from L and S; X1 is any amino acid selected from C, G, S, L, N, D, F, I, V, Y, Q, K, E, M, and T; X2 is any amino acid selected from L, Q, N, D, Y, R, C, G, S, F, T, I, V, A, K, and M; X3 is any amino acid selected from P, N, T, I, S, M, Q, R, L, G, V, A, E, D, Y, F, and H; X4 is any amino acid selected from K, N, S, I, M, E, Q, L, V, A, H, D, Y, and F; and X5 is any amino acid selected from M and Y;
preferably, the sequence of the Fc monomer further comprises an amino acid sequence set forth in SEQ ID NO: 2.

The IL-15 receptor α sushi domain in the present invention means that the extracellular domain of IL-15 receptor α starts from cysteine residue (C1) encoded by a first exon 2 and ends at cysteine residue (C4) encoded by a fourth exon 2, and both residues C1 and C4 are contained in the sushi domain. The amino acid sequences of IL-15 receptor α sushi domains formed by substitution, deletion or addition of one or more amino acid residues and having corresponding activities are also included in the present invention.

In a preferred embodiment of the above long-acting interleukin-15 fusion protein, the sequence of the IL-15 receptor α sushi domain comprises an amino acid sequence set forth in SEQ ID NO: 3; preferably, the sequence of the IL-15 functional fragment comprises an amino acid sequence set forth in SEQ ID NO: 4.
SEQ ID NO: 1 (amino acid sequence of monomeric Fc) 
APELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALP
APIEKTISKAKGQPREPQVYTX0PPSRDELTKNQVSLX1CX2VKGFYPSDIAVE
WESNGQPENNYKTTX3PVLDSDGSFFLYSX4LTVDKSRWQQGNVFSCSVX5H
EALHNHYTQKSLSLSPGK.
SEQ ID NO: 2 (amino acid sequence of monomeric Fc) 
APELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALP
APIEKTISKAKGQPREPQVYTSPPSRDELTKNQVSLRCHVKGFYPSDIAVEWES
NGQPENNYKTTKPVLDSDGSFFLYSDLTVDKSRWQQGNVFSCSVYHEALHN
HYTQKSLSLSPGK.
SEQ ID NO: 3 (amino acid sequence of IL-15RαSu) 
ITCPPPMSVEHADIWVKSYSLYSRERYICNSGFKRKAGTSSLTECVLNKA
TNVAHWTTPSLKCIR.
SEQ ID NO:4 (amino acid sequence of IL-15 mutant)

NWVNVISDLKKIEDLIQSMHIDATLYTESDVHPSCKVTAMKCFLLELQVI
SLESGDASIHDTVENLIILANDSLSSNGNVTESGCKECEELEEKNIKEFLQSFV
HIVQMFINTS.

The IL-15 functional fragment in the present invention refers to a cytokine of about 12-14 kD discovered by Grabstein *et al.* in 1994 (J G Giri et al., EMBO J. 1994 Jun 15; 13(12): 2822-2830.), which can function in the normal immune response of the body, e.g., to promote the proliferation of T cells, B cells and NK cells. The amino acid sequences of IL-15 functional fragments formed by substitution, deletion or addition of one or more amino acid residues and having corresponding activities are also included in the present invention.

In a second aspect of the present invention, provided is a method for constructing the above long-acting interleukin-15 fusion protein, comprising:
(a) linking an Fc monomer to an IL-15 receptor α sushi domain via a linker peptide segment;
(b) obtaining an IL-15 functional fragment; and
(c) subjecting (a) and (b) to co-transfection expression, or to separate expression followed by protein assembly *in vitro.*

Co-transfection expression refers to mixing a vector having an insert for an Fc monomer and an IL-15 receptor α sushi domain, which are linked via a linker peptide segment, with a vector for an IL-15 functional fragment at an appropriate ratio to transfect cells (such as Expi293 cells), which are subjected to culturing, expression and purification (such as one-step purification with protein G) to obtain an IL-15 fusion protein.

The method of protein assembly *in vitro* refers to transfecting cells (such as Expi293 cells) separately with a vector for an Fc monomer and an IL-15 receptor α sushi domain, which are linked via a linker peptide segment, and a vector for an IL-15 functional fragment at an appropriate ratio, culturing and inducing these cells, mixing the supernatants of the induced cells at an appropriate ratio, and performing purification (such as one-step purification with protein G) to obtain a high-purity target protein.

In a third aspect of the present invention, provided is a nucleic acid molecule encoding the above long-acting interleukin-15 fusion protein.

In a fourth aspect of the present invention, provided is a plasmid containing the above nucleic acid molecule. Regulatory sequences, such as promoters and enhancers, are operably linked.

In a fifth aspect of the present invention, provided is a host cell containing the above plasmid. The host cell of the present invention may be any prokaryotic cell or eukaryotic cell, including but not limited to bacterial cells (such as *Escherichia coli* and *Bacillus subtilis*), insect cells (e.g., by using a baculovirus expression system), and yeast or mammalian cells (such as CHO or BHK cell lines). Other suitable host cells are known to those skilled in the art.

In a sixth aspect of the present invention, provided is a pharmaceutical composition containing an effective prophylactic or therapeutic dose of the above long-acting interleukin-15 fusion protein, the above nucleic acid molecule, or the above plasmid, and a pharmaceutically acceptable carrier. The composition includes but is not limited to a freeze-dried dosage form, an aqueous solution dosage form, a liposome, a capsule dosage form, etc. The concentration of the fusion protein of the present invention or the nucleic acid molecule or plasmid thereof may vary from about 0.1% to 100% (by weight).

In a seventh aspect of the present invention, provided is a detection kit containing the above long-acting interleukin-15 fusion protein, the above nucleic acid molecule, or the above plasmid; preferably, the detection kit is used for detecting pathogens and tumor cells. The pathogens include viruses, bacteria, fungi, parasitic infections, etc. The tumor cells include various benign tumor cells, malignant tumor cells (i.e., cancer cells), solid tumor cells, and hematogenous carcinoma cells.

The beneficial effects of the present invention lie in that the present invention provides a method for diagnosing, preventing or treating a disease, comprising administering the IL-15 fusion protein, nucleic acid molecule, plasmid, and pharmaceutical composition of the present invention to a subject.

The disease described in the present invention is an autoimmune disease, an inflammatory disease, a neurodegenerative disease, a cancer, or a pathogen infection.

Preferably, the IL-15 fusion protein of the present invention can be used for treating a cancer. The cancers in the present invention include, but are not limited to, lymphoma, blastomas, sarcomas (including liposarcoma), neuroendocrine tumors, mesothelioma, schwannomas, meningiomas, adenomas, melanomas, and aleukemic leukemia or lymphatic malignant tumors. More specific example of that above cancers include squamous cell carcinoma (e.g., squamous epithelial cell carcinoma), lung cancer, small cell lung cancer, non-small cell lung cancer, lung adenocarcinoma and lung squamous cell carcinoma, peritoneal cancer, hepatocellular carcinoma, gastric cancer, gastrointestinal cancer, pancreatic cancer, malignant glioma, cervical cancer, ovarian cancer, liver cancer, bladder cancer, hepatocellular carcinoma, breast cancer, colon cancer, rectal cancer, colorectal cancer, endometrial or uterine cancer, salivary gland cancer, renal cancer, prostate cancer, vulvar cancer, thyroid cancer, liver cancer, anal cancer, penile cancer, testicular cancer, esophageal cancer, bile duct tumor, head cancer, neck cancer, bone marrow stromal tumor, osteoclast tumor, multiple myeloma, osteolytic bone cancers, central nervous system tumor, brain tumors (glioma, neuroblastoma, astrocytoma, medulloblastoma, ependymoma, and retinal neuroblastoma), nasopharyngeal carcinoma, basal cell carcinoma, cholangiocarcinoma, Kaposi's sarcoma, primary liver cancer or endometrial cancer, and tumors of the vascular system (angiosarcoma and hemangiopericytoma).

Preferably, the IL-15 fusion protein of the present invention can be used for treating pathogen infection. The pathogens in the present invention include but are not limited to bacteria, fungi, viruses and parasites.

The conditions that can be treated by the IL-15 fusion protein of the present invention specifically include, but are not limited to, congestive heart failure (CHF), vasculitis, rosacea, acne, eczema, myocarditis and other myocardial conditions, systemic lupus erythematosus, diabetes, spondylopathy, synovial fibroblast proliferation, bone loss, Paget's disease, disuse osteopenia, malnutrition, periodontal disease, familial spleen anemia, Langerhans cell histiocytosis, spinal cord injury, acute septic arthritis, osteomalacia, hypercortisolism, monostotic fibrous dysplasia, multiple osteofibrous dysplasia, periodontal reconstruction and fracture, sarcoidosis, bone metastasis/osteodynia treatment and malignant hypercalcemia of body fluids, ankylosing spondylitis and other spinal arthropathies, transplant rejection, viral infection, hematological tumors, Hodgkin's lymphoma, non-Hodgkin's lymphoma (Burkitt's lymphoma, small lymphocytic lymphoma/chronic lymphocytic leukemia, mycosis fungoides, mantle cell lymphoma, follicular lymphoma, diffuse giant B-cell lymphoma, marginal zone lymphoma, hairy cell leukemia and lymphoplasmacytic leukemia), lymphocyte precursor cell tumor, B-cell acute lymphoblastic aleukemic leukemia/lymphoma, T-cell acute lymphoblastic aleukemic leukemia/lymphoma, thymoma, mature T and NK cell tumors, peripheral T cell aleukemic leukemia, mature T cell aleukemic leukemia/T cell lymphoma, large granular lymphocytic leukemia, Langerhans cell histiocytosis, myeloma of acute myelogenous leukemia, mature acute myelogenous leukemia (AML), differentiation acute myelogenous leukemia, acute promyelocytic leukemia, acute myelomonocytic leukemia, acute monocytic leukemia, spinal dysplasia syndrome, chronic myeloproliferative disease, chronic myeloid leukemia, osteoporosis, hepatitis, HIV, AIDS, spinal arthritis, rheumatoid arthritis, inflammatory bowel disease (IBD), sepsis and septic shock, cicatrizing enteritis, psoriasis, scleroderma, graft versus host disease (GVHD), allogenic islet graft rejection, hematological malignancies such as multiple myeloma (MM), myelodysplastic syndrome (MDS) and acute myelogenous leukemia (AML), tumor-related inflammations, peripheral nerve injury, or demyelinating diseases.

The IL-15 fusion protein, nucleic acid molecule, plasmid, and pharmaceutical composition of the present invention can be administered to human or animal subjects via various routes of administration, usually depending on the characteristics of the disease to be treated. Generally, any medically acceptable mode of administration, including oral, rectal, topical, intraocular, intracisternal, intracerebroventricular, intratracheal, intranasal drip, transdermal, subcutaneous, intrathecal, intramuscular, abdominal cavity, intraperitoneal, intracranial infusion or intravenous infusion administration, can be used to implement the method of the present invention.

### Brief Description of the Drawings

Fig. 1 shows a structural model diagram of a novel long-acting interleukin-15 fusion protein;
Fig. 2 shows a diagram of the reduced SDS-PAGE analysis of the novel long-acting interleukin-15 fusion protein and staining with Coomassie brilliant blue;
Fig. 3 shows high-performance liquid chromatography (HPLC) analysis of the novel long-acting interleukin-15 fusion protein, wherein the analytes are FL115-2a (C) and FL115-2b (D), and the control proteins are sFc (A) and Fc (b);
Fig. 4 shows that the biological activity of the novel long-acting interleukin-15 fusion protein is evaluated by a proliferation assay on CTLL-2, wherein the positive control is a fusion protein of IL-15 and IL-15 receptor α (positive control);
Fig. 5 shows the *in vivo* anti-tumor effect of the novel long-acting interleukin-15 fusion protein FL115-2a in mice;
Fig. 6 shows the effect of the novel long-acting interleukin-15 fusion protein of the present invention on the body weight of mice subcutaneously inoculated on the back with B16F10 melanoma tumor;
Fig. 7 shows the effect of the novel long-acting interleukin-15 fusion protein of the present invention on the survival rate of mice subcutaneously inoculated on the back with B16F10 melanoma tumor;
Fig. 8 shows the anti-tumor effect of the novel long-acting interleukin-15 fusion protein in mice and the effect thereof on the body weight of the mice subcutaneously inoculated on the back with B16F10 melanoma tumor, wherein the positive control is an Fc fusion protein of IL-15 (abbreviated as PC), IL15 KIH (Fc fusion protein of IL-15&IL15α) and WT-IL15, and the negative control is PBS;
Fig. 9 shows the anti-tumor effect of the novel long-acting interleukin-15 fusion protein *in vivo* on 5 mice in each group;
Fig. 10 shows the anti-tumor effect of the novel long-acting interleukin-15 fusion protein on the treatment of each of the mice in each group. The tumor weight of each mouse was weighed, and whether there was a statistical difference between groups was analyzed by one-way ANOVA analysis and statistics;
Fig. 11 shows a decreased number of Treg cells *in vivo* in mice inoculated with B16F10 melanoma tumor after receiving the novel long-acting interleukin-15 fusion protein, as analyzed by flow cytometry, which is specifically evidenced by the decrease of Foxp3+CD25+;
Fig. 12 shows an increased number of NK cells *in vivo* and increased secretion of IFN-γ and perforin in mice inoculated with B16F10 melanoma tumor after receiving the novel long-acting interleukin-15 fusion protein, as analyzed by flow cytometry; and
Fig. 13 shows increased IL6 *in vivo* in mice inoculated with B16F10 melanoma tumor after receiving the novel long-acting interleukin-15 fusion protein, as analyzed by flow cytometry.

### Detailed Description of Embodiments

In order to make those of skill in the art better understand the solution of the present application, the technical solutions in the embodiments of the present application will be described below clearly and completely in conjunction with instances. Obviously, the described embodiments are only some, rather than all, of the embodiments of the present application. Based on the embodiments in the present application, all other embodiments obtained by those of ordinary skill in the art without involving any inventive effort shall fall within the scope of protection of the present application.

In order to understand the present invention more thoroughly, some definitions are listed below. The above definitions are intended to include grammatical equivalents.

The term fusion protein has two different meanings. One is the expression product of recombined two genes obtained by DNA recombination technology, and the other is a group of proteins that mediate the fusion of two cell plasma membranes, such as one of the two glycoproteins contained in the outer leaflet of Sendai virus lipid bilayer, which mediates the action of fusion between the virus envelope and the plasma membrane of the host cell. Another glycoprotein is hemagglutinin ceramidase. Two different proteins can be linked to form a macromolecule either by a chemical method or by gene fusion.

IL-15 is a soluble cytokine, which acts as a chemokine of various immune cells to participate in and regulate the inflammatory response and immune response in the body. The biological function thereof is similar to that of IL-2, which is produced by a variety of cells.

As used herein, the term "monomer" means a molecule that can undergo polymerization to provide structural units for the basic structure of a polymer. The process in which a large number of monomers combine to form a polymer is called polymerization.

As used herein, the terms "Fc" and "Fc monomer" include polypeptides comprising antibody constant regions other than the first constant regions of immunoglobulin domains. Therefore, Fc refers to the last two constant regions of IgA, IgD and IgG immunoglobulin domains, the last three constant regions of IgE and IgM immunoglobulin domains, and the flexible hinges connecting the N-termini of these domains. For IgA and IgM, Fc can comprise J chain. For IgG, Fc comprises immunoglobulin domains Cy2 and Cy3 and a hinge between Cγ1 and Cγ2. Although the boundary of the Fc region may vary, the Fc region of human IgG heavy chain is usually defined as containing residue C226 or P230 at its carboxyl terminal, in which the numbering method is in accordance with EU index of Kabat. Fc can refer to the region that has been isolated, or the region in the context of antibody, antibody fragment or Fc fusion. Fc may be an antibody, an Fc fusion, or a protein or protein domain containing Fc. Particularly preferred are Fc variants, which are non-naturally occurring Fc variants obtained by synthetic biology.

Functional fragments are a class of compounds, the molecular structures of which are between amino acids and proteins and which have strong biological activities *per se.*

As used herein, the term "N-terminal", also known as amino terminal, NH2-terminal, N-terminal or amine terminal, is the start of a protein or polypeptide and refers to the free amine group (-NH2) located at the terminal of a polypeptide. The term "C-terminal", also known as carboxyl terminal, carboxyl group terminal, C-terminal tail, C-terminal or COOH-terminal, is the terminal of a protein or polypeptide and is terminated by a free carboxyl (-COOH) terminal.

As used herein, the term "amino acid" means one of the 20 naturally occurring amino acids or any unnatural analogues, which can be located in a specified position. The term "protein" herein means at least two covalently linked amino acids, including proteins, polypeptides, oligopeptides and peptides. Proteins can be composed of naturally occurring amino acids and peptide bonds, or the structure of synthetic peptide mimetics, which are called "analogues". Therefore, the term "amino acid" or "peptide residue" as used herein means naturally occurring and synthetic amino acids. For example, for the purpose of the present invention, homophenylalanine, citrulline and norleucine are considered as amino acid for the purpose of the present invention. The term "amino acid" also includes imino acid residues such as proline and hydroxyproline. The side chain can be in (R) or (S) configuration. In a preferred embodiment, the amino acid is present in (S) or L-configuration. If non-naturally occurring side chains are used, non-amino acid substitutions can be used, for example, to prevent or delay *in vivo* degradation.

As used herein, the term "nucleic acid" means a polymer composed of nucleotide units (ribonucleotides, deoxynucleotides, related naturally occurring structural variants and their synthetic non-naturally occurring analogues) via phosphodiester bonds. Therefore, the term includes nucleotide polymers, in which nucleotides and bonds therebetween include non-naturally occurring synthetic analogues, such as but not limited to, thiophosphates, aminophosphates, methylphosphates, chiral methylphosphates, 2'-O-methylribonucleotides, peptide nucleic acids (PNAs), etc. For example, these polynucleotides can be synthesized using an automatic DNA synthesizer. The term "oligonucleotide" generally refers to a short polynucleotide, usually no more than about 50 nucleotides. It should be understood that when the nucleotide sequence is represented by a DNA sequence (i.e., A, T, G, and C), this also includes an RNA sequence in which "T" is replaced by "U" (i.e., A, U, G, and C).

Herein, conventional symbols are used to describe nucleotide sequences: the left-hand end of a single-stranded nucleotide sequence is the 5' end; and the left-hand direction of a double-stranded nucleotide sequence is called 5' direction. The direction in which 5' to 3' nucleotides are added to the newborn RNA transcript is called the transcription direction. A DNA strand with the same sequence as mRNA is called a coding strand.

As used herein, the term "encoding" means the inherent property of a specific nucleotide sequence in a polynucleotide, such as a gene, a cDNA or an mRNA, to serve as a template for the synthesis of other polymers and macromolecules by biological processes with either a defined nucleotide sequence or a defined amino acid sequence, and the biological properties resulting therefrom. Therefore, if the transcription and translation of mRNA produced from this gene result in a protein in cells or other biological systems, the gene encodes the protein. A coding strand, the nucleotide sequence of which is identical to mRNA and is usually provided in a sequence listing, and a non-coding strand, used as a transcription template, for a gene or a cDNA, can be referred to as encoding the protein or other product of that gene or cDNA. Unless otherwise specified, the term "nucleotide sequence encoding an amino acid sequence" includes all nucleotide sequences which are degenerate with each other and encode the same amino acid sequence. A nucleotide sequence that encodes a protein or an RNA may also include introns.

As used herein, the term "plasmid" means a plasmid artificially constructed on the basis of a natural plasmid to adapt to laboratory operations. A nucleic acid molecule can be introduced into a host cell, thereby producing a transformed host cell. A vector may include a nucleic acid sequence that allows it to replicate in a host cell, such as origin of replication, and may also include one or more selectable marker genes and other genetic elements known in the art.

As used herein, the term "host cell", also called recipient cell, refers to a host cell that receives a foreign gene during transformation and transduction (infection).

As used herein, the term "pharmaceutically acceptable carrier" means a conventional pharmaceutically acceptable carrier. Remington's Pharmaceutical Sciences, EW Martin, Mack Publishing Co., Easton, Pa., 15th edition (1975), describes compositions and preparations suitable for drug delivery of one or more therapeutic compounds or molecules (such as one or more antibodies), and additional agents.

As used herein, the "diagnosis" of a disease refers to the diagnosis of a condition and the development thereof in a patient after examination. The "prevention" of a disease refers to inhibiting the complete development of the disease. The term "treatment" refers to therapeutic intervention to improve the signs or symptoms of a disease or a pathological condition after it begins to develop.

The term "administration" herein means selecting an appropriate route to introduce the substance into a subject. For example, if the selected route is intravenous, the composition is administered by introducing the substance into the vein of the subject.

The term "effective prophylactic/therapeutic dose" herein means an amount of a specific agent sufficient to achieve a desired effect in a subject treated with the agent. An exact dosage will depend on the purpose of treatment and can be determined by those skilled in the art by using well-known techniques. The dosage range can be 0.01-100 mg/kg body weight or more, such as 0.1, 1, 10, or 50 mg/kg body weight, preferably 1-10 mg/kg. As is well known in the art, in terms of the degradation of an antibody or Fc fusion, systemic or local drug delivery and new protease synthesis rate, as well as age, body weight, general health, sex, diet, administration time, drug interaction, and the severity of the disease, adjustment may be necessary and can be determined by those skilled in the art by means of conventional experimental methods. Such agents include the monomeric Fc domain molecules described herein. In one non-limiting example, this may be the amount of an HIV-specific monomeric Fc domain (or HIV-specific CH3 domain molecule) for preventing, treating or ameliorating HIV infection. Ideally, a therapeutically effective amount of antibody is an amount sufficient to prevent, treat or ameliorate an infection or disease, such as that ascribable to HIV infection in a subject, without causing significant cytotoxic effects in the subject. A therapeutically effective amount of an agent for preventing, ameliorating and/or treating a subject will depend on the subject being treated, the type and severity of the indisposition, and the mode of administration of a therapeutic composition.

The term "autoimmune disease" herein refers to a disease in which the immune system generates an immune response (e.g., a B cell or T cell response) against some antigens (i.e., autoantigens) of a normal host and then causes damage to tissues. The autoantigen may be derived from a host cell, or may be derived from a symbiotic organism, such as a microorganism (called a symbiotic organism) that normally colonizes the mucosal surface. Autoimmune diseases affecting mammals include but are not limited to rheumatoid arthritis, juvenile pauciarthritis, collagen-induced arthritis, adjuvant-induced arthritis, Sjögren's syndrome, multiple sclerosis, experimental autoimmune encephalomyelitis, inflammatory bowel disease (e.g., Crohn's disease and ulcerative colitis), autoimmune gastric atrophy, pemphigus vulgaris, psoriasis, vitiligo, type 1 diabetes, non-obese diabetes, myasthenia gravis, Graves' disease, Hashimoto's thyroiditis, sclerosing cholangitis, sclerosing sialadenitis, systemic lupus erythematosus, autoimmune thrombocytopenic purpura, Goodpasture's syndrome, Addison's disease, systemic sclerosis, polymyositis, dermatomyositis, autoimmune hemolytic anemia, pernicious anemia, etc.

The term "virus" herein is from, but is not limited to, the following families of viruses: the Retroviridae family (e.g., human immunodeficiency virus (HIV) and human T cell leukemia virus (HTLV)); the Picornaviridae family (e.g., poliovirus, hepatitis A virus, hepatitis C virus, enterovirus, human coxsackie virus, rhinovirus, echovirus, and foot-and-mouth disease virus); the Calmyxoviridae family (such as virus strains that cause gastroenteritis); the Togaviridae family (e.g., equine encephalitis virus and rubella virus); the Flaviviridae family (e.g., dengue virus, yellow fever virus, West Nile virus, St. Louis encephalitis virus, Japanese encephalitis virus, and other encephalitis viruses); the Coronaviridae family (e.g., coronavirus and severe acute respiratory syndrome (SARS) virus); the Rhabdoviridae family (e.g., vesicular stomatitis virus and rabies virus); the Paramyxoviridae family (e.g., parainfluenza virus, mumps virus, measles virus, and respiratory syncytial virus (RSV)); the Orthomyxoviridae family (e.g., influenza virus); the Bunyaviridae family (e.g., Hantavirus, Sin Nombre virus, Rift Valley fever virus, bunya virus, phleboviruses, and Nairo virus); the Arenaviridae family (e.g., hemorrhagic fever virus, Machupo virus, and Junin virus); the Reoviridae family (e.g., reovirus, orbiviurse, and rotavirus); the Birnaviridae family; the Hepadnaviridae family (e.g., hepatitis B virus); the Parvoviridae family (e.g., parvovirus); the Papovaviridae family (e.g., papillomavirus, polyoma virus, and BK virus); the Adenoviridae family (e.g., most adenoviruses, such as adeno-associated viruses); the Herpesviridae family (e.g., herpes simplex virus (HSV-1 and HSV-2), cytomegalovirus (CMV), Epstein-Barr virus (EBV), varicella zoster virus (VZV), and other herpes viruses, including HSV-6); the Poxviridae family (e.g., smallpox virus, vaccinia virus, and poxvirus); the Iridoviridae family (such as African swine fever virus); the Viraceae family (e.g., Ebola virus and Marburg virus); the Caliciviridae family (e.g., Norwalk virus); and unclassified viruses (e.g., the pathogen of spongiform encephalopathy, the pathogen of hepatitis delta (considered as a defective satellite of hepatitis B virus), and astroviruses).

The term "bacteria" herein is from, but not limited to, *Helicobacter pylori, Borelia burgdorferi, Legionella pneumophila,* mycobacteria (such *as M. tuberculosis, M. avium, M. intracellulare, M. kansaii,* and *M. gordonae), Staphylococcus aureus, Neisseria gonorrhoeae, Neisseria meningitidis, Listeria monocytogenes, Streptococcus pyogenes* (group A streptococcus), *Streptococcus agalactiae* (group B streptococcus), *Streptococcus* (the Viridans group streptococci), *Streptococcus faecalis, Streptococcus bovis, Streptococcus* (anaerobic bacteria), *Streptococcus pneumoniae,* pathogenic *Campylobacter, Enterococcus, Haemophilus influenzae, Bacillus anthracis, Corynebacterium diphtheriae, Corynebacterium,* classical swine fever virus, *Clostridium perfringens, Clostridium tetani, Enterobacter aerogenes, Klebsiella pneumoniae, Pasteurella multocida, Bacteroides, Clostridium, Streptomyces maltophilia, Treponema pallidum, Treponema, Leptospira,* or *Actinomyces israelli.*

The term "fungus" herein is from, but not limited to, *Cryptococcus neoformans, Histoplasma capsulatum, Coccidioides immitis, Blastomyces dermatitidis, Chlamydia trachomatis,* or *Candida albicans.*

The term "parasite" herein is from, but not limited to, *Plasmodium falciparum* or *Toxoplasma gondii.*

The term "cancer" herein is a solid tumor or a hematogenous carcinoma. The solid tumor described in the present invention is sarcoma or cancer, such as fibrosarcoma, myxosarcoma, liposarcoma, chondrosarcoma, osteogenic sarcoma, or another sarcoma, synovioma, mesothelioma, Ewing's tumor, leiomyosarcoma, rhabdomyosarcoma, colon cancer, lymphatic malignancy, pancreatic cancer, breast cancer, lung cancer, ovarian cancer, prostate cancer, hepatocellular carcinoma, squamous cell carcinoma, basal cell carcinoma, adenocarcinoma, hidradenocarcinoma, sebaceous gland carcinoma, papillary carcinoma, papillary adenocarcinoma, medullary carcinoma, bronchial carcinoma, renal cell carcinoma, hepatocellular carcinoma, cholangiocarcinoma, choriocarcinoma, nephroblastoma, cervical carcinoma, testicular tumor, bladder cancer, or central nervous system tumor (such as glioma, astrocytoma, medulloblastoma, craniopharyngioma, ependymoma, pineal gland, hemangioblastoma, acoustic neuroma, oligodendroglioma, hemangioma, melanoma, neuroblastoma, or retinoblastoma). The hematogenous carcinoma described in the present invention is leukemia, such as acute leukemia (such as acute lymphocytic leukemia, acute myelogenous leukemia, acute myeloid leukemia, and myeloblastic, promyelocytic, myelomonocytic, monocytic and erythroleukemia); and chronic leukemia (such as chronic myelocytic (granulocytic) leukemia, chronic granulocytic leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia, or myelodysplasia.

Unless otherwise specified, all the technical and scientific terms used herein have the same meaning as commonly understood by those of ordinary skill in the field to which the present disclosure belongs. Unless otherwise explicitly specified in the context, the singular terms "a", "an", and "the" include plural indicators. It is to be understood that all base sizes or amino acid sizes given for nucleic acids or polypeptides, as well as all molecular weights or molecular weight values, are approximate and provided for description. Although methods and materials similar or equivalent to those described herein can be used in the practice or testing of the present disclosure, suitable methods and materials are described below. The term "comprise" means "include". All publications, patent applications, patents and other references mentioned herein are incorporated by reference in their entirety. In case of conflict, the present description (including the explanation of terms) shall prevail. In addition, the materials, methods and examples are only illustrative and not restrictive.

The standard recombinant DNA technologies and molecular cloning technologies used in the examples are well known in the art (Ausubel, F. M. et al., Current Protocols in Molecular Biology, published by Greene Publishing Assoc. and Wiley-Interscience), and the materials and methods suitable for microbial growth are well known in the art. Main chemical and biochemical reagents are purchased from KAPA Biosystems, New England Biolabs, TransGen Biotech, Thermo Fisher Scientific, OMEGA bio-tek, etc.

The present invention will be illustrated in detail below in conjunction with specific examples.

### Example 1. Design and preparation of novel long-acting interleukin-15 fusion protein

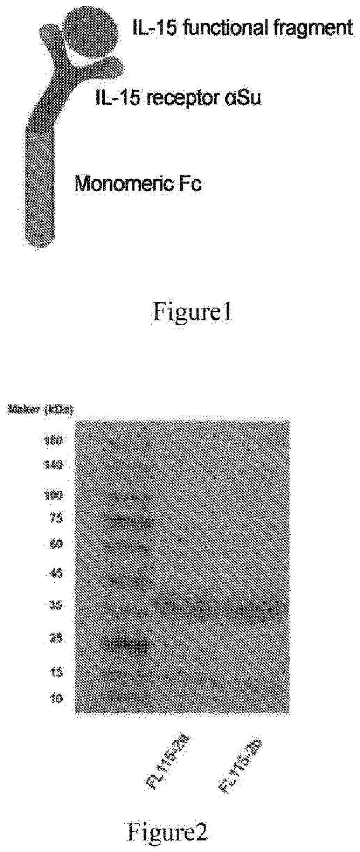

A model diagram of the designed novel long-acting interleukin-15 fusion protein was shown in Fig. 1. Genes encoding IL15Rα-GGGGS-sFc (SEQ ID NO: 5), IL15Rα-(GGGGS)₃-sFc (SEQ ID NO: 6), IL15Rα-Fc (SEQ ID NO: 7), and IL15(N72D) (the sequence was SEQ ID NO: 4) were synthesized by Genescript, Nanjing. The genes were cloned into eukaryotic expression vector PTT (purchased from Thermo Fisher). The successfully constructed plasmid was subjected to maxi-preparetion and then transiently co-transformed into Expi293 cells (purchased from Thermo Fisher) for expression (wherein IL15Rα-(GGGGS)₃-sFc was co-transformed with IL15(N72D) to obtain FL115-2a, IL15Rα-GGGGS-sFc was co-transformed with IL15(N72D) to obtain FL115-2b, and IL15Rα-Fc was co-transformed with IL15(N72D) to obtain an Fc fusion protein of IL-15), and purification was carried out by means of protein G resin (purchased from GE Healthcare) (for specific transformation, expression and purification methods, see: YING, Tianlei et al., JOURNAL OF BIOLOGICAL CHEMISTRY, 2012, 287(23): 19399-19408). The purity of the purified protein was verified by SDS-PAGE gel, and the results showed that the purity of the protein was good, and the band of interest was correct, without unwanted bands (see Fig. 2).

### Example 2. Physicochemical properties of novel long-acting interleukin-15 fusion protein

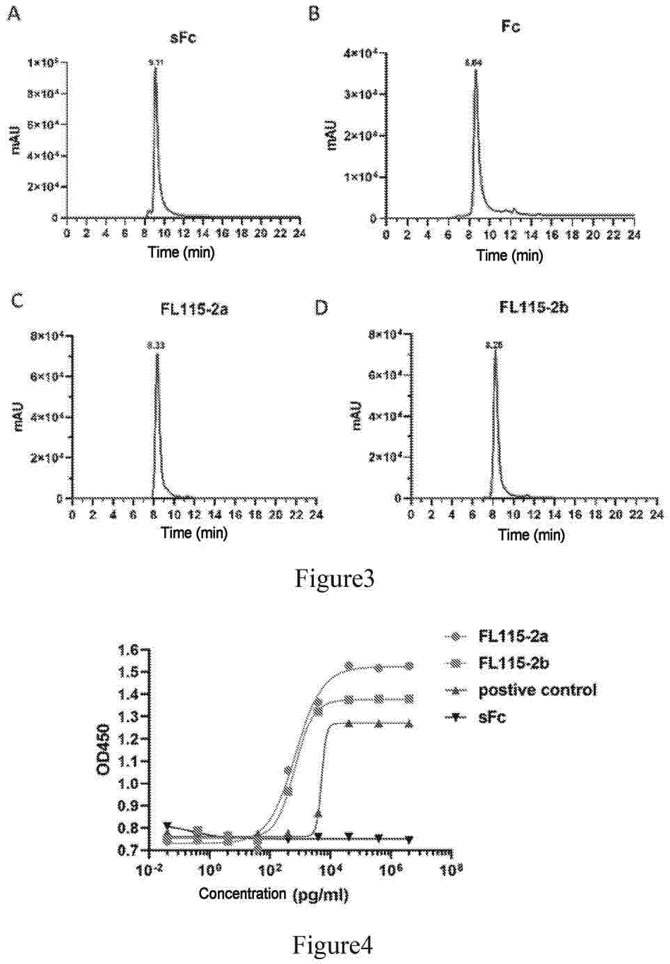

The novel long-acting interleukin-15 fusion proteins (FL115-2a and FL115-2b) were expressed and purified according to the method of Example 1. In addition, the Fc fusion protein of IL-15 was designed and constructed in the same way, and the expression and purification of the Fc fusion protein were carried out through the same expression system and the same manner to obtain the related protein. Purification was carried out by means of protein G resin (for specific transformation, expression and purification methods, see: YING, Tianlei et al., JOURNAL OF BIOLOGICAL CHEMISTRY, 2012, 287(23): 19399-19408), the yield of the novel long-acting interleukin-15 fusion protein was up to 10 mg/L, which was obviously advantageous relative to the yield of the Fc fusion protein of IL-15 of 1 mg/L and theoretically had a significant advantage in terms of industrial production costs. In addition, we took an equal volume of 100 ul of the above protein for high-performance liquid chromatography (HPLC) to verify the homogeneity of the protein. As shown in Fig. 3, the novel long-acting interleukin-15 fusion protein was homogeneous in nature and existed in a monomeric form. FL115-2a and FL115-2b represented the linkage between sFc and IL15Rα via GGGGS and (GGGGS)₃ peptide segments, respectively. The above results indicated that the novel long-acting interleukin-15 fusion protein had potentially better developability.

### Example 3. Evaluation of biological activity of novel long-acting interleukin-15 fusion protein in CTLL-2 cell line

In order to evaluate the biological activity of the novel long-acting interleukin-15 fusion protein, a proliferation assay on CTLL-2 cell line was used (Santos-Savio A. et al., Biotecn Aplic. 2000, 17:221-4). According to the procedure described below, mitochondrial staining with 3-(4,5-dimethylthiazol-2-yl)-2,5-diphenyltetrazolium bromide (MTT) (purchased from Sigma) was used (for the specific method, see: Mossman T.J. Immunol. Methods. 1983, 65(1-2): 55-63), and the biological activity was measured by stimulating the proliferation of these cells. In a 96-well culture plate (purchased from Costar, USA), serial dilutions of FL115-2a, FL115-2b, positive control (IL15&IL15Rα) and sFc were carried out in a volume of 50 µL of an RPMI medium (purchased from Gibco) supplemented with 10% fetal bovine serum (FBS) (purchased from Gibco) and 50 µg/mL of gentamicin (purchased from Sigma), starting from a concentration of 4 µg/mL. CTLL-2 cells (purchased from ATCC) were washed five times with RPMI medium in advance and added to a plate in a volume of 50 µL at 5×103 cells/well. Incubation was carried out at 37°C, 5% CO2 and 98% relative humidity for 72 h. Cell viability was determined by MTT staining. sFc was not biologically active because it did not induce the proliferation of CTLL-2 cells, and this was different from FL115-2a, FL115-2b and positive control (IL15&IL15Rα), which stimulated proliferation in a dose-dependent manner (see Fig. 4).

### Example 4. Pharmacodynamic verification of novel long-acting interleukin-15 fusion protein in vivo

In order to assess the pharmacodynamics of the novel long-acting interleukin-15 fusion protein, we injected 5×105 B16F10 mouse melanoma cells into 6-8-week-old female C57BL/6 mice (purchased from Jiangsu GemPharmatech Co., Ltd.) at the right back. On Days 1 and 8 after inoculation, various doses of FL115-2a-1, positive control (IL15&IL15Rα), wild-type IL15 (WT-IL15) or negative control (PBS) were given. The corresponding FL115-2a-2 was given every other day after tumor inoculation. The tumor size in the mice was measured on the specified date. The experimental result was average plus or minus standard deviation, with 5 mice in each group. The statistical significance of the experimental data was analyzed by one-way ANOVA, with ^{∗∗} representing p < 0.01. The positive control was the fusion protein of IL-15 and IL-15 receptor α (positive control), and the WT-IL15 and PBS groups were controls; and FL115-2a-1 represented administration on Days 1 and 8, and FL115-2a-2 represented administration every other day.

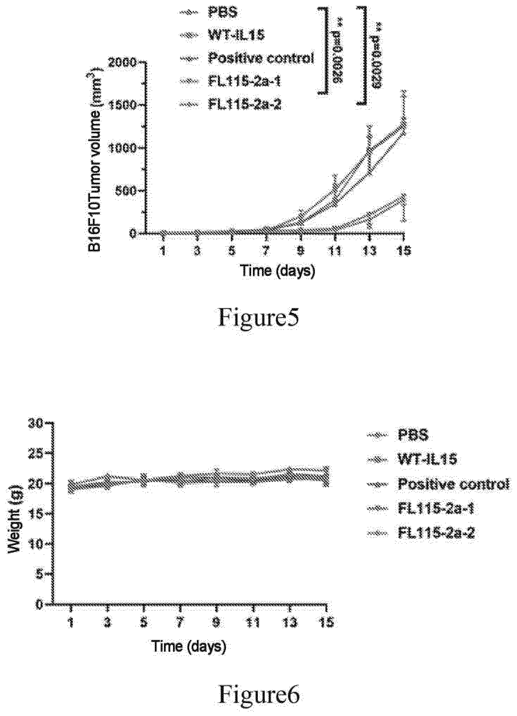

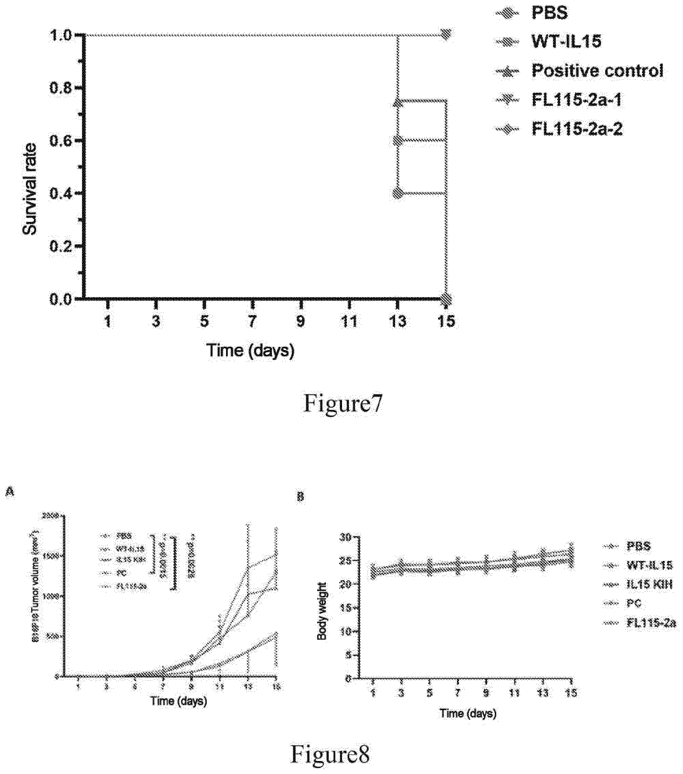

The experimental results showed that the modes of administration of FL115-2a-1 and FL115-2a-2 did not affect the anti-tumor efficacy of the novel long-acting interleukin-15 fusion protein *in vivo,* and the two groups had very obvious anti-tumor efficacy, with a statistically significant difference from that of the negative control group. We suspected that it was related to the prolonged half-life of IL-15 *in vivo,* as shown in Fig. 5. In addition, we tested the body weight of the mice in each group and confirmed that the safety of the fusion protein was good, as shown in Fig. 6, and FL115-2a-1 and FL115-2a-2 groups showed good survival rates, as shown in Fig. 7.

### Example 5. Novel long-acting interleukin-15 fusion protein had efficient anti-tumor activity in vivo

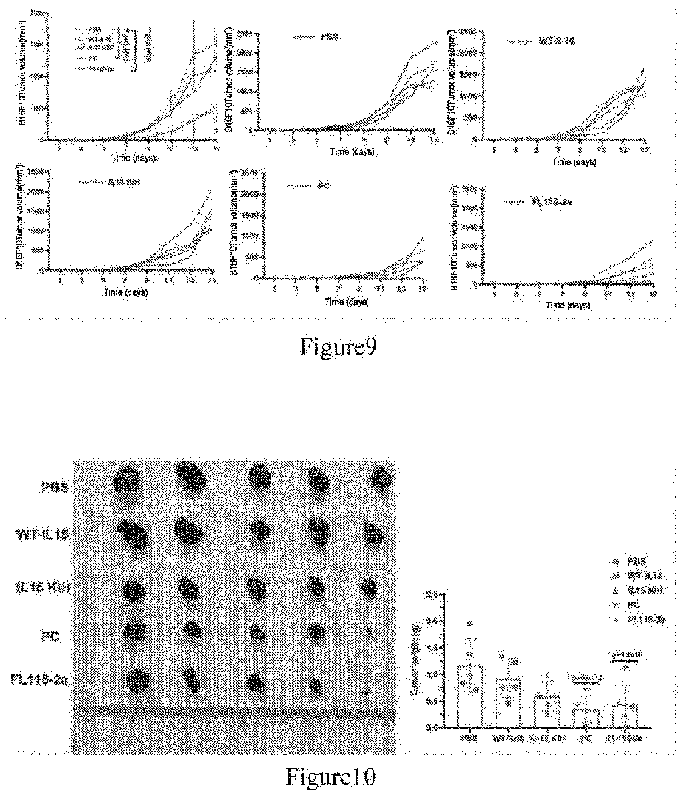

In order to assess the anti-tumor effect of the novel long-acting interleukin-15 fusion protein *in vivo,* we established a mouse transplanted tumor model by subcutaneously transplanting B16F10 mouse melanoma cells into 6-8-week-old female C57BL/6 mice (purchased from Jiangsu GemPharmatech Co., Ltd.) on the back according to the experimental design of the pharmacodynamics verification above. After the model was established, FL115-2a, PC (Fc fusion protein of IL-15), IL15KIH (Fc fusion protein of IL-15&IL15Rα), WT-IL15 and negative control PBS were injected into the tail vein on Days 1 and 8, and the tumor size in the mice was measured every other day. FL115-2a exhibited an *in vivo* tumor growth inhibition effect comparable to PC and had a statistically significant difference from the negative control group, as shown in Fig. 8. The body weight of mice in each group was not affected, indicating the *in vivo* safety of the novel long-acting interleukin-15 fusion protein in mice, as shown in Fig. 8. In addition, we also analyzed the tumor inhibitory effects respectively in the 5 mice in each group and found that the tumor inhibitory effects in the mice in the FL115-2a group were consistent, and the anti-tumor inhibitory effect was obvious, with an inhibition rate of up to 62%, as shown in Fig. 9. We anatomized the mice, took out the tumor of each mouse and weighed the tumor. FL115-2a had an obvious tumor inhibitory effect in mice due to its efficient anti-tumor activity *in vivo,* and presented a significant difference from the negative control group in terms of whether the size or weight, as shown in Fig. 10.

### Example 6. Analysis of cell phenotype by flow cytometry

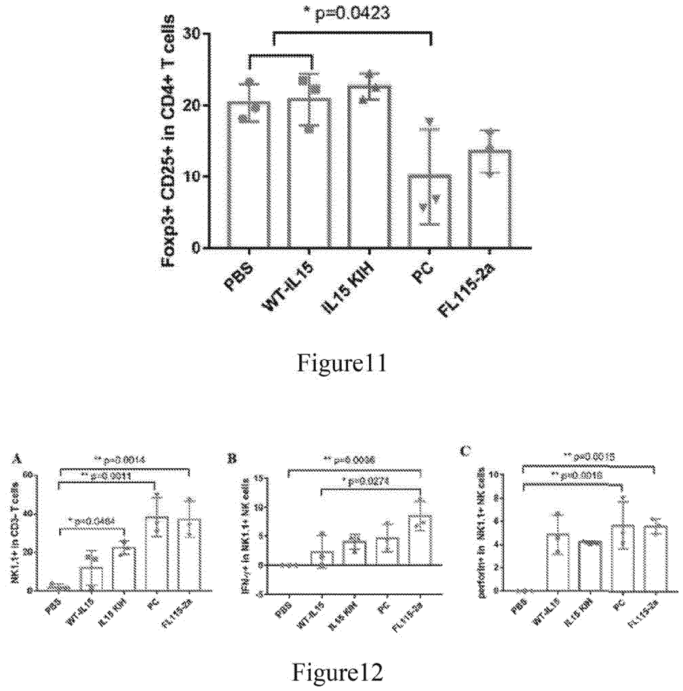

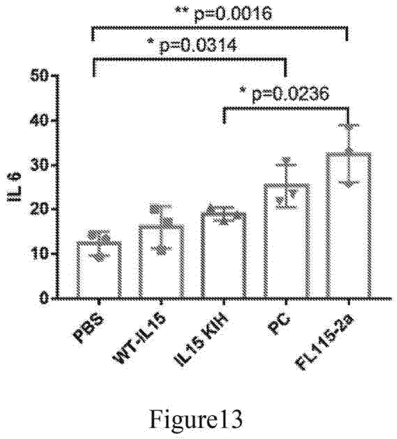

After anesthesia, peripheral venous blood was collected from the orbital veins of mice, and anticoagulation and erythrocyte lysis treatments were performed immediately. After the mice were executed humanely, the spleen was taken out and spleen cells were collected immediately, and after filtration through a 70 uM nylon sieve (purchased from FALCON) and erythrocyte lysis treatment, a spleen single cell suspension was obtained. In addition, the tumor tissue was taken out, the structure of the tumor tissue was gently destroyed with tweezers, and the tumor tissue was then digested with 0.2 mg/ml Collagenase IV (purchased from Sigma-Aldrich) and 0.1 mg/ml DNAse I (purchased from Sigma-Aldrich) at 37°C for 15 min. The single cell suspension was collected and the remaining tumor tissue was digested with the above enzyme solution for 25 min. The combined single cell suspension was filtered through a 70 micron nylon mesh. Peripheral blood single cells, spleen cells and tumor cells were stained with a flow antibody (from BD), then fixed with a 4% paraformaldehyde solution (from Sigma-Aldrich) and stored in the dark or immediately loaded onto a flow cytometer (from BD) for cell phenotype detection. After staining with the antibody, by flow cytometry analysis, it could be found that the number of Treg cells in the tumor tissue of the mice treated with FL115-2a was decreased, which was specifically evidenced by the decrease of Foxp3+CD25+, as shown in Fig. 11. In addition, the number of NK cells and the secretion of IFN-γ and perforin were increased. The experimental results suggested that the novel long-acting interleukin-15 fusion protein could not only reduce the number of Treg cells and its inhibitory effect, but could also promote the capability of NK cells to infiltrate tumor tissues to a larger extent and significantly increase the secretion of IFN-γ and perforin, which further suggested that the novel long-acting interleukin-15 fusion protein could effectively activate the immune system to kill tumor cells and improve the tumor inhibitory effect of IL-15 *in vivo,* as shown in Fig. 12. In addition, flow cytometry also showed that the novel long-acting interleukin-15 fusion protein could increase the expression of IL-6, as shown in Fig. 13.

The above description of the disclosed embodiments enables those skilled in the art to implement or use the present invention. The above description is only for the preferred embodiments of the present invention, and it should be pointed out that those skilled in the art can also make several improvements and supplements without departing from the principles of the present invention, and these improvements and supplements should also be regarded as being in the scope of protection of the present invention. Many modifications to these embodiments will be obvious to those skilled in the art, and the general principles defined herein can be implemented in other embodiments without departing from the spirit or scope of the present invention. Therefore, the present invention will not be limited by the embodiments shown herein, but conforms to the widest scope consistent with the principles and novel features disclosed herein.

## Claims

1. A long-acting interleukin-15 fusion protein, comprising an Fc monomer, an IL-15 receptor α sushi domain, and an IL-15 functional fragment, wherein one terminal of the IL-15 receptor α sushi domain is linked to the Fc monomer, and the other terminal of the IL-15 receptor α sushi domain is linked to the IL-15 functional fragment.

2. The long-acting interleukin-15 fusion protein according to claim 1, wherein the C-terminal of the IL-15 receptor α sushi domain is linked to the Fc monomer, and the IL-15 receptor α sushi domain is linked to the IL-15 functional fragment via a covalent bond.

3. The long-acting interleukin-15 fusion protein according to claim 2, wherein the Fc monomer is linked to the IL-15 receptor α sushi domain via a linker peptide, wherein the linker peptide is GGGGS or (GGGGS)₃.

4. The long-acting interleukin-15 fusion protein according to any of claims 1-3, wherein the sequence of the Fc monomer comprises an amino acid sequence set forth in SEQ ID NO: 1, wherein
X0 is any amino acid selected from L and S; X1 is any amino acid selected from C, G, S, L, N, D, F, I, V, Y, Q, K, E, M, and T; X2 is any amino acid selected from L, Q, N, D, Y, R, C, G, S, F, T, I, V, A, K, and M; X3 is any amino acid selected from P, N, T, I, S, M, Q, R, L, G, V, A, E, D, Y, F, and H; X4 is any amino acid selected from K, N, S, I, M, E, Q, L, V, A, H, D, Y, and F; and X5 is any amino acid selected from M and Y

5. The long-acting interleukin-15 fusion protein according to claim 4, wherein the sequence of the Fc monomer further comprises an amino acid sequence set forth in SEQ ID NO: 2.

6. The long-acting interleukin-15 fusion protein according to any of claims 1-5, wherein the sequence of the IL-15 receptor α sushi domain comprises an amino acid sequence set forth in SEQ ID NO: 3; and the sequence of the IL-15 functional fragment comprises an amino acid sequence set forth in SEQ ID NO: 4.

7. A method for constructing the long-acting interleukin-15 fusion protein according to any of claims 1-6, comprising:
(a) linking an Fc monomer to an IL-15 receptor α sushi domain via a linker peptide segment;
(b) obtaining an IL-15 functional fragment; and
(c) subjecting (a) and (b) to co-transfection expression, or to separate expression followed by protein assembly *in vitro.*

8. A nucleic acid molecule encoding any of the long-acting interleukin-15 fusion protein according to any of claims 1-7.

9. A plasmid containing the nucleic acid molecule according to claim 8.

10. A host cell containing the plasmid according to claim 9.

11. A pharmaceutical composition containing an effective prophylactic or therapeutic dose of any of the long-acting interleukin-15 fusion protein according to claims 1-6, the nucleic acid molecule according to claim 8 or the plasmid according to claim 9, and a pharmaceutically acceptable carrier.

12. A detection kit containing the long-acting interleukin-15 fusion protein according to any of claims 1-6, the nucleic acid molecule according to claim 8 or the plasmid according to claim 9.

13. The detection kit according to claim 12, wherein the detection kit is used for detecting pathogens and tumor cells.
